# EUROPÄISCHE PATENTSCHRIFT

(11) **EP 1 372 502 B1**
(45) Veröffentlichungstag und Bekanntmachung des Hinweises auf die Patenterteilung: **16.04.2008**
(21) Anmeldenummer: 02718105.6
(22) Anmeldetag: 07.02.2002
(51) Int. Cl.: A61B 17/70, A61B 17/86

(54) **VERANKERUNGSELEMENT**
ANCHOR ELEMENT
ELEMENT D'ANCRAGE

(30) Priorität: 27.03.2001 DE 10115014
(43) Veröffentlichungstag der Anmeldung: 02.01.2004
(73) Patentinhaber: BIEDERMANN MOTECH GmbH, 78054 VS-Schwenningen (DE)
(72) Erfinder: BIEDERMANN, Lutz, 78048 VS-Villingen (DE); HARMS, Jürgen, 76227 Karlsruhe (DE)
(74) Vertreter: Hofer, Dorothea
(86) Internationale Anmeldenummer: PCT/EP2002/001284
(87) Internationale Veröffentlichungsnummer: WO 2002/076314

(56) Entgegenhaltungen:
- EP-A- 0 933 065
- WO-A-01/03593
- WO-A-94/00066
- FR-A- 2 720 923
- FR-A- 2 786 088

## Beschreibung

Die Erfindung betrifft ein Verankerungselement mit einer einen Gewindeabschnitt und einen als kugelsegmentförmigen Abschnitt ausgebildeten Kopf aufweisenden Schraube und einem Aufnahmeteil zum Verbinden der Schraube mit einem Stab nach dem Oberbegriff des Patentanspruches 1. Ein derartiges Verankerungselement wird insbesondere in der Wirbelsäulenchirurgie, aber auch in der Unfallchirurgie bei anderen Knochen eingesetzt.

Ein solches Verankerungselement ist beispielsweise aus der DE 43 07 576 C1 bekannt. Bei derartigen bekannten Verankerungselementen und Schrauben sind der Gewindeabschnitt der Schraube und ihr Kopf einstückig ausgebildet. Da der Chirurg verschiedenste Längen der Schrauben benötigt, muß er immer verschiedene Sätze solcher Schrauben vorrätig haben. Das macht ein erhebliches Vorratslager notwendig mit der Folge erheblicher Kosten.

Aus der WO 01/03593 A1 ist ein Verankerungselement bekannt, welches eine Schraube und ein Aufnahmeteil zum Verbinden der Schraube mit einem Stab aufweist. Das Aufnahmeteil weist einen ersten Bereich mit einem im Wesentlichen U-förmigen Querschnitt zur Aufnahme des einzusetzenden Stabs und an seinem anderen Ende einen Bereich zur Aufnahme des Kopfs auf. Ferner ist ein auf den Stab Druck ausübendes Element in Form einer Kappe, die auf das Aufnahmeteil aufgeschraubt wird, vorgesehen. Die Schraube hat einen Gewindeabschnitt und einen Kopf. Der Kopf der Schraube ist zweiteilig ausgebildet und weist ein schraubenschaftseitiges Unterteil und ein endständiges Oberteil auf. Das Unterteil hat einen verbreiterten Kragen oder Rand und das Oberteil ist einseitig kugelsegmentförmig ausgeführt.

Aufgabe der Erfindung ist es, diesen Nachteil zu beseitigen.

Diese Aufgabe wird durch das in Patentanspruch 1 gekennzeichnete Verankerungselement gelöst. Dadurch wird es möglich, daß der Chirurg bei der Anwendung den Gewindeabschnitt vor oder nach dem Implantieren auf eine gewünschte Länge kürzt und dann diesen mit dem Kopf und dem Aufnahmeteil verbindet. Auf diese Weise wird die Vorratshaltung wesentlich verkleinert, und gleichzeitig erhöhen sich die Möglichkeiten für den Chirurgen, feinere Justierungen vorzunehmen, da die Schrauben auf jedes Längenmaß kürzbar sind.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Seitenansicht einer ersten Ausführungsform in geschnittener Darstellung;
- Fig. 2: die in Fig. 1 gezeigte Ausführungsform in Explosionsdarstellung;
- Fig. 3: eine entsprechende Explosionsdarstellung einer zweiten Ausführungsform;
- Fig. 4: eine Seitenansicht der ersten in den beiden Ausführungsformen verwendeten Knochenschraube;
- Fig. 5: eine Draufsicht auf die Knochenschraube in Fig. 4;
- Fig. 6: eine Seitenansicht einer zweiten Ausführungsform der in den ersten beiden Ausführungsbeispielen verwendeten Knochenschraube;
- Fig. 7: eine Draufsicht auf die in Fig. 6 gezeigte Knochenschraube;
- Fig. 8: eine Seitenansicht einer dritten Ausführungsform einer in den ersten beiden Ausführungsbeispielen gezeigten Knochenschraube;
- Fig. 9: eine Seitenansicht einer weiteren Ausführungsform in Schnittdarstellung;
- Fig. 10: eine Seitenansicht einer weiteren Ausführungsform in Schnittdarstellung; und
- Fig. 11: eine vergrößerte Darstellung des Details X von Fig. 10.

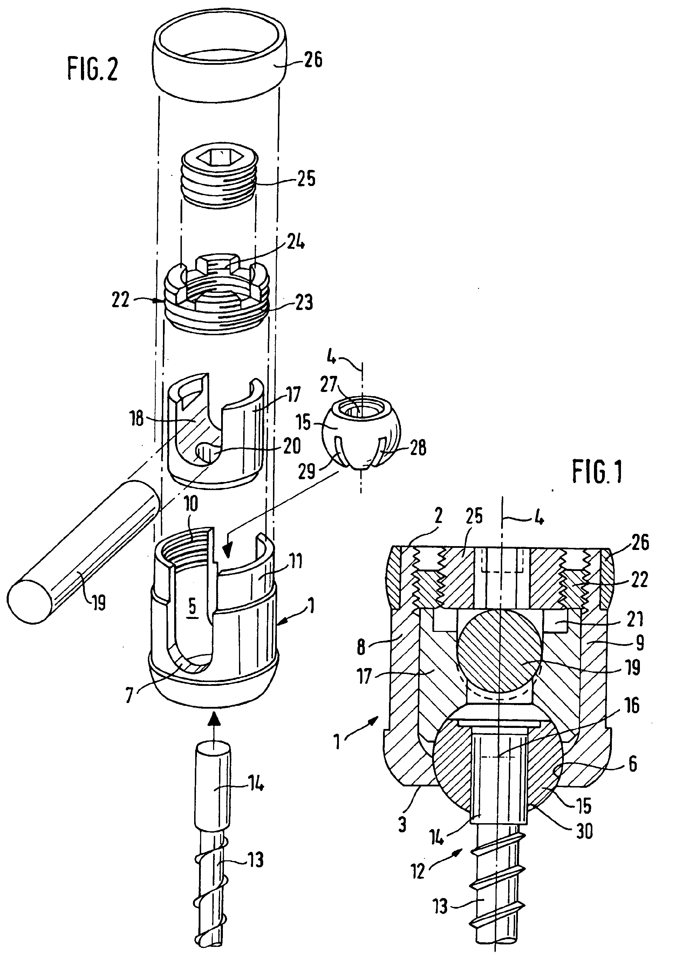

Bei der in den Fig. 1 und 2 gezeigten ersten Ausführungsform weist das Verankerungselement ein zylindrisch ausgebildetes Aufnahmeteil 1 mit einem ersten Ende 2 und einem gegenüberliegenden zweiten Ende 3 auf. Die beiden Enden erstrecken sich senkrecht zu einer Symmetrie- bzw. Längsachse 4. Koaxial zu der Längsachse 4 ist eine von dem ersten Ende 2 aus sich erstreckende erste koaxiale Bohrung 5 vorgesehen, die sich bis zu einem vorbestimmten Abstand von dem zweiten Ende 3 hin erstreckt. An dem zweiten Ende 3 ist eine zweite Bohrung vorgesehen, deren Durchmesser kleiner als der Durchmesser der ersten Bohrung ist. In dem gezeigten Ausführungsbeispiel ist die zweite Bohrung als eine Öffnung ausgebildet, deren Rand als hohlkugelsegementförmiger Abschnitt geformt ist, dessen Mittelpunkt zum ersten Ende 2 hin gerichtet ist.

Das Aufnahmeteil 1 weist ausgehend von dem ersten Ende 2 eine sich senkrecht zur Längsachse 3 erstreckende U-förmige Ausnehmung 7 auf mit zwei zum ersten Ende 2 hin endenden freien Schenkeln 8, 9. Angrenzend an das erste Ende 2 weisen die Schenkel ein Innengewinde 10 auf. Der Grund der U-förmigen Ausnehmung erstreckt sich bis zu einem vorgegebenen Abstand von dem zweiten Ende 3 hin. Angrenzend an das erste Ende 2 weisen die Schenkel 8, 9 außen einen Abschnitt 11 auf, dessen Außendurchmesser kleiner ist als der Außendurchmesser des angrenzenden Abschnittes des Aufnahmeteiles.

Die mit dem Aufnahmeteil 1 zusammenwirkende Schraube 12 weist einen als Knochenschraube ausgebildeten Gewindeabschnitt 13 und einen in der in Fig. 1 gezeigten montierten Darstellung damit verbundenen kugelsegmentförmigen Kopf 15 auf. Der Kopf weist einen Radius auf, der so bemessen ist, daß der Kopf bei der in Fig. 1 gezeigten Aufnahme des Kopfes 15 in der zweiten Bohrung 6 zu einem dort gebildeten hohlkugelsegementförmigen Wandabschnitt paßt, wobei der hohlkugelsegmentförmige Abschnitt so ausgebildet ist, daß der Mittelpunkt 16 der Kugel so weit zum ersten Ende 2 hin versetzt ist, daß der Abschnitt ein Widerlager bildet und die Kugel bzw. der Kopf 15 in dem hohlkugelsegmentförmigen Abschnitt der zweiten Bohrung 6 gehalten wird.

Es ist ferner ein Druckelement 17 vorgesehen, welches zylindrisch ausgebildet ist und einen Außendurchmesser aufweist, der gerade so groß ist, daß das Druckelement in die erste Bohrung 5 einführbar und in dieser in Axialrichtung hin- und herbewegbar ist. Auf seiner, dem zweiten Ende 3 zugewandten Unterseite weist das Druckelement 17 einen zur Längsachse 4 symmetrisch ausgebildeten hohlkugelsegmentförmigen Abschnitt auf, dessen Radius dem Radius des Kopfes 5 entspricht. Das Druckelement weist eine sich quer zur Längsachse 4 erstreckende U-förmige Ausnehmung 18 auf, deren freie Schenkel sich zum ersten Ende 2 hin erstrecken. Der seitliche Durchmesser dieser U-förmigen Ausnehmung ist so gewählt, daß ein aufzunehmender Stab 19 in die Ausnehmung einsetzbar ist und in dieser seitlich geführt ist. Die Tiefe der hohlkugelsegmentförmigen Ausnehmung ist so gewählt, daß sie in einem Abstand von dem zweiten Ende 3 endet, der größer ist als der dem Radius des Kopfes 15 entsprechenden Abstandes von dem Mittelpunkt 16 zum ersten Ende 2 hin gesehen. Am Grund der U-förmigen Ausnehmung 18 schließt sich eine koaxiale Bohrung 20 an, deren Durchmesser kleiner als der Durchmesser des aufzunehmenden Stabes 19 ist.

Wie aus Fig. 1 ersichtlich ist, weist die U-förmige Ausnehmung 18 an ihrem zum ersten Ende 2 hin gerichteten Ende einen Abschnitt 21 auf, dessen innere Weite größer als der Durchmesser der U-förmigen Ausnehmung 18 ist.

Auf der dem ersten Ende 2 zugewandten Seite schließt sich an das Druckelement 17 eine Mutter 22 an, die ein zu dem Innengewinde 10 passendes Außengewinde 23 und zusätzlich ein Innengewinde 24 aufweist. Die Innenabmessung der Mutter 22 ist so gewählt, daß die innere Weite kleiner als der Durchmesser des Abschnittes 21 ist und größer als der Durchmesser des Stabes 19 und damit der U-förmigen Ausnehmung 18 ist. Ferner ist eine Innenmutter 25 mit einem zum Innengewinde 24 passenden Außengewinde vorgesehen. Schließlich ist eine das freie Ende angrenzend an das erste Ende 2 umfassende Hülse 26 vorgesehen, die im zusammengesetzten Zustand auf dem ringförmigen Abschnitt 11 aufsitzt, wie dieser in Fig. 1 gezeigt ist.

Wie am besten aus Fig. 2 zu ersehen ist, weist die Mutter 22 einen Schlitz und die Innenmutter 25 eine Sechskantöffnung zum jeweiligen getrennten Angreifen von Schraubendrehern auf.

Wie am besten aus Fig. 2 zu ersehen ist, ist der Kopf 15 als eine an ihrem dem ersten Ende 2 zuzuwendenden Ende abgeflachte Kugel ausgebildet und weist eine zu der Längsachse 4 koaxiale Bohrung 27 auf. Der Durchmesser der Bohrung 27 ist gleich dem Außendurchmesser des Schaftes 14 und so ausgebildet, daß der Schaft in die Bohrung reibschlüssig einschiebbar ist. Wie aus Fig. 2 ersichtlich ist, ist das so geformte hohlkugelsegmentförmige Element auf seiner dem abgeflachten Ende gegenüberliegenden Seite mit in Umfangsrichtung einen Abstand zueinander aufweisenden und sich parallel zur Längsachse 4 erstreckenden und bis zu dem der abgeflachten Seite gegenüberliegenden Ende reichenden Ausschnitten 28, 29 versehen. Dadurch wird erreicht, daß der dem ersten Ende 2 abgewandte Rand 30 zum Einführen des Schaftes 14 federnd nach außen nachgebbar ausgebildet ist.

Im Betrieb wird zunächst die Schraube 12 in den Knochen bzw. den Wirbel eingeschraubt. Zu diesem Zweck weist der Schaft 14 bekannte Eingriffsmöglichkeiten wie etwa einen Innensechskant auf. Dann kürzt der Operateur den Schaft 14 auf die gewünschte Länge und setzt zunächst das Aufnahmeteil mit der zweiten Bohrung auf den Schaft 14 und führt dann den Kopf von dem ersten Ende 2 her auf den Schaft 14, so daß der Schaft 14 vom federnd nachgebenden Rand 30 her in die Bohrung 27 eingeführt wird und der Kopf den Schaft in der in Fig. 1 gezeigten Weise einschließt. Der Kopf 15 und der Schaft 14 sind reibschlüssig miteinander verbunden. Anschließend wird das Druckelement 17 eingesetzt und durch Einschrauben der Mutter 22 so auf den Kopf 15 gedrückt, daß dieser eine gewünschte Drehstabilisierung erfährt. Die Hülse 26 wird aufgebracht und dann wird mit Hilfe der Innenmutter 25 der Stab 19 fixiert. Der Stab 19 übt über das Druckelement 17 einen zusätzlichen Druck auf den Kopf 15 aus.

Durch den vom ersten Ende 2 her gesehen ausgeübten Druck auf den Kopf 15 wird der geschlitzte Kopf 15 einerseits mit dem Schaft 14 bewegungsfest verbunden bzw. verklemmt, gleichzeitig wird der Kopf in seiner Drehstellung arretiert.

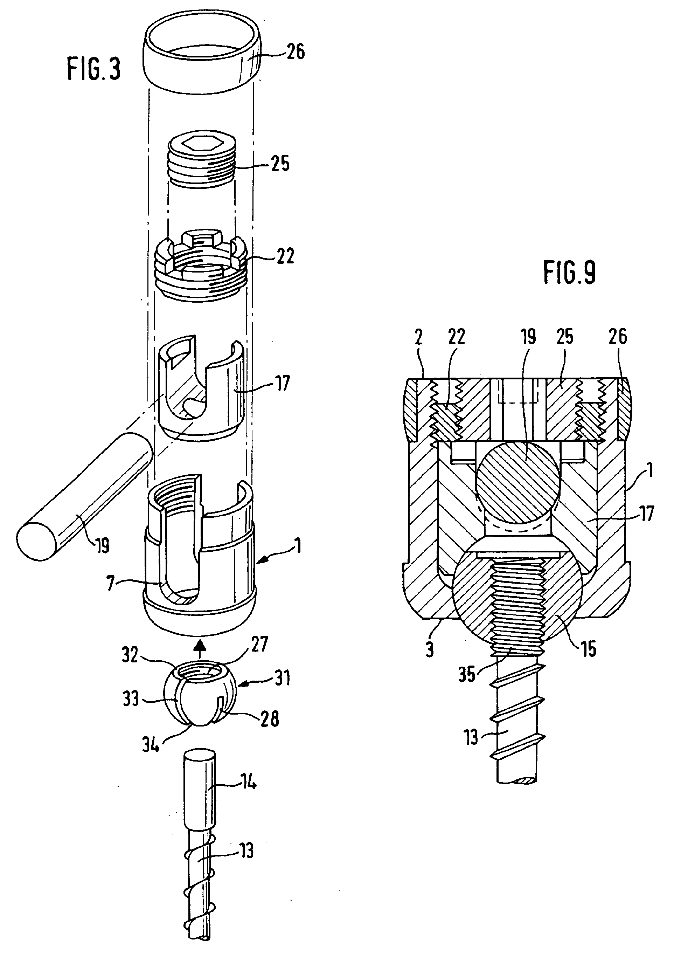

Die in Fig. 3 gezeigte zweite Ausführungsform unterscheidet sich von der zuvor beschriebenen Ausführungsform durch einen abgewandelten Kopf 31. Dieser weist wie bei der ersten Ausführungsform in Umfangsrichtung zueinander versetzte Ausschnitte 28 auf, die an dem dem ersten Ende abgewandten Rand 34 frei enden und einen Abstand von dem dem ersten Ende 2 zugewandten Rand 32 aufweisen. Es ist jedoch ein Ausschnitt 33 vorgesehen, der sich vollständig von dem Rand 32 bis zum gegenüberliegenden Rand 34 erstreckt, mit der Folge, daß das so gebildete Kugelsegment um ein durch die Breite des Ausschnittes 33 bestimmtes Ausmaß zusammendrückbar ist. Die Breite des so gebildeten Schlitzes 33 ist so gewählt, daß der Kopf 31 zunächst so weit zusammendrückbar ist, daß er in der in Fig. 3 gezeigten Richtung vom zweiten Ende 2 her in die erste Bohrung 5 hineindrückbar ist und daß dann der Schaft 14 in den Kopf in der gleichen Weise wie oben beschrieben einschiebbar ist und auf die gleiche Weise in verklemmter Position gehalten wird.

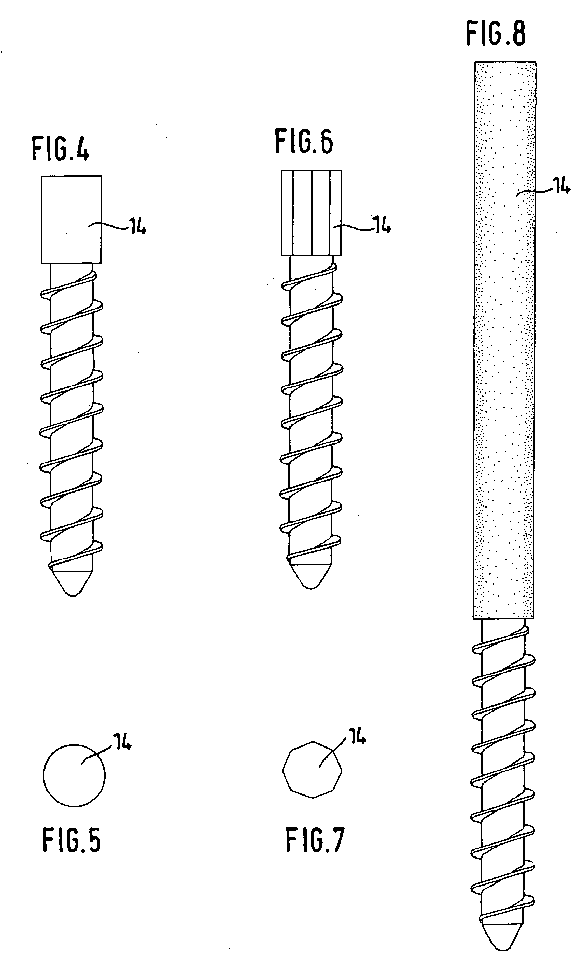

Der Schaft 14 der Schraube hat vorzugsweise die in den Fig. 4 und 5 gezeigte zylindrische Form oder auch eine in den Fig. 6 und 7 gezeigte mehreckige Form. In letzterer ist der Querschnitt achteckig ausgebildet. Eine weitere bevorzugte Ausführungsform ist in Fig. 8 gezeigt. Der Schaft ist hier zylindrisch ausgebildet und weist eine rauhe Oberfläche auf, die das Ineingriffbringen zwischen Kugel 15 und Schaft erleichtert.

Die in Fig. 9 gezeigte weitere Ausführungsform stimmt in allen Merkmalen, die das Aufnahmeteil 1, das Druckelement 17, den Stab 19 und die Schrauben 22 und 25 betreffen, mit den vorhergehenden Ausführungsbeispielen überein. Der einzige Unterschied besteht darin, daß der Kopf 15 als ein Kugelsegment ausgebildet ist, welches in seinen äußeren Abmessungen den beiden vorhergehenden Kugelsegmenten entspricht, jedoch keine Ausschnitte 28 oder 33 aufweist. Statt dessen weist das Kugelsegment auf der Innenseite seiner Bohrung 27 ein Innengewinde auf. Anstelle des Schaftes 14 ist ein Schaft 35 mit einem Gewinde vorgesehen, welches zu dem Innengewinde des Kopfes passend ausgebildet ist. Die Bohrung ist als eine Sackbohrung ausgebildet, die an dem dem freien Ende 2 zugewandten Ende endet oder dort einen Anschlag aufweist, so daß die Schraube nur so weit in die gezeigte Stellung einschraubbar ist, in der sie nicht aus dem Kugelsegment an dessen abgeflachter Seite heraussteht. Wie in Fig. 9 gezeigt ist, sind das Innengewinde des Kopfes 15 und das entsprechende Außengewinde des Schaftes 35 in der Richtung vorzugsweise entgegengesetzt zur Richtung des Gewindes des Gewindeabschnittes 13 der Knochenschraube ausgebildet.

Der Betrieb erfolgt in gleicher Weise wie bei dem zuerst beschriebenen Ausführungsbeispiel, wobei nach dem Kürzen des Schaftes 35 der Kopf 15 vom ersten Ende 2 des Aufnahmeteils 1 her in die Bohrung 5 eingeführt und auf den vom zweiten Ende 3 her eingeführten Schaft 35 aufgeschraubt wird.

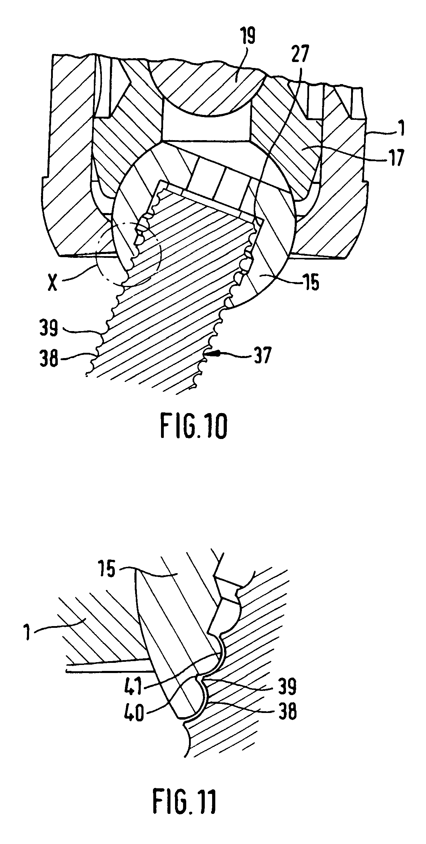

Die in den Figuren 10 und 11 gezeigte weitere Auführungsform stimmt in allen Merkmalen, die das Aufnahmeteil 1, das Druckelement 17, den Stab 19 und die Schrauben 22 und 25 betreffen, mit den vorhergehenden Ausführungsbeispielen überein. Anstelle des Schafts 35 und des Kopfes 15 der Ausführungsform nach Fig. 9, welche die zusammenwirkenden Gewinde aufweist, ist bei dieser Ausführungsform der Schaft 37 in einem Abschnitt angrenzend an das dem Knochengewindeabschnitt gegenüberliegenden Ende als Wellenstab ausgebildet. Die Außenfläche des Schaftes weist in Umfangsrichtung verlaufende Wellentäler 38 und dazwischenliegende Wellenkämme 39 auf. Die Wellentäler 38 haben in Umfangsrichtung gesehen einen kreisabschnittförmigen Querschnitt und ihr Durchmesser auf halber Höhe bzw. Tiefe ist deutlich größer als der entsprechende Durchmesser des Wellenbergs 39, so dass die Wellenberge 39 spitz sind im Verhältnis zu dem Grund der Wellentäler 38. Der Kopf 15 ist als ein Kugelsegment ausgebildet, welches in seinen äußeren Abmessungen den zuvor genannten Kugelsegmenten entspricht, das jedoch keine Ausschnitte 28 oder 33 aufweist. Auf der Innenseite seiner Bohrung 27 weist das Kugelsegment des Kopfes 15 in Umfangsrichtung verlaufende Wellen mit Wellentälern 40 und Wellenkämmen 41 auf, die jeweils den Wellenkämmen 39 bzw. den Wellentälern 38 des Schaftes 37 entsprechen. Zwischen den Wellentälern 38 und den Wellenkämmen 39 des Schaftes einerseits und den entsprechenden Wellenkämmen 40 und Wellentälern 41 andererseits ist ein kleiner Zwischenraum, so daß der Schaft in das Kugelsegment einführbar ist.

Der Betrieb erfolgt in ähnlicher Weise wie bei dem Ausführungsbeispiel gemäß Fig. 9. Das Kürzen des gewellten Schaftes 37 ist jedoch bei dieser Ausführungsform einfacher als das Kürzen des Schaftes 35 mit dem Gewinde gemäß Fig. 9, da die Wellentäler 38 ein leichtes Abschneiden ermöglichen, während bei dem Schaft 35 mit dem Gewinde gemäß Fig. 9 darauf geachtet werden muß, daß das Gewinde nicht zerstört wird. Nach dem Kürzen des Schaftes 37 wird der Kopf 15 von dem ersten Ende 2 des Aufnahmeteils 1 in die Bohrung 5 eingeführt und auf den Schaft 37 aufgedrückt. Dabei wirken die Wellen des Schaftes 37 und die entsprechenden der Bohrung 27 des Kopfes 15 zusammen, so daß der Schaft gehalten wird.

In den oben beschriebenen Ausführungsbeispielen wird der Kopf 15 jeweils durch einen mit dem Aufnahmeteil 1 einstückig ausgebildeten Rand gehalten. Ein solches Widerlager kann auch auf andere Weise gebildet werden, beispielsweise ist es möglich, die erste Bohrung 5 vollständig durch das Aufnahmeteil 1 hindurch zu bohren und dann angrenzend an das zweite Ende ein den Kopf 15 aufnehmendes Halteelement einzuspannen.

In den oben beschriebenen Ausführungsbeispielen weist das Aufnahmeteil stets die Mutter 22 und eine Innenmutter 25 sowie eine Hülse 26 auf. Diese Fixierung kann in bekannter Weise auch anders ausgebildet sein. Insbesondere kann gegebenenfalls auch lediglich eine Innenmutter vorgesehen sein.

Bei dem oben unter Bezugnahme auf Fig. 9 beschriebenen Ausführungsbeispiel weist der Kopf 15 keine Ausschnitte 28, 33 auf. In einer weiteren Ausführungsform weisen Kopf 15 und Schaft 35 wie bei der in Fig. 9 gezeigten Darstellung zueinander passende Gewinde auf. Der Kopf 15 weist jedoch zusätzlich den sich über die gesamte Länge erstreckenden Ausschnitt 33, so daß wie bei der in Fig. 3 gezeigten Ausführungsform der Kopf ohne eingeschraubten Schaft von dem Rand 3 aus durch Zusammendrücken in das Aufnahmeteil einschiebbar ist und dann auf den ebenfalls vom Ende 3 her einführbaren Schaft 35 durch Einschrauben aufnimmt und mit diesem verbunden wird. Der Schlitz führt beim Aufsetzen der Druckelemente bzw. beim Ausüben des Druckes auf den Kopf 15 dazu, daß gleichzeitig der Kopf und der Schaft 35 fester zusammengedrückt werden als ohne einen solchen Schlitz.

In einer weiteren Ausführungsform können zusätzlich noch Ausschnitte 28 in der in Fig. 3 gezeigten Weise vorgesehen sein, um so ein noch stärkeres Anpressen an den Gewindeschaft 35 zu bewirken.

## Patentansprüche

1. Verankerungselement mit einer **einen Schaft** mit Gewindeabschnitt (13) und einen als kugelsegmentförmiger Abschnitt ausgebildeten Kopf (15) aufweisenden Schraube (12) und einem Aufnahmeteil (1) zum Verbinden der Schraube (12) mit einem Stab (19), wobei das Aufnahmeteil (1)
ein erstes Ende (2) und ein diesem gegenüberliegendes zweites Ende (3), eine durch die beiden Enden (2, 3) gehende Längsachse (4),
eine zur Längsachse (4) koaxiale Bohrung (5), einen in einem an das erste Ende (2) angrenzenden ersten Bereich mit einem im wesentlichen U-förmigen Querschnitt (7) mit zwei freien ein Gewinde aufweisenden Schenkeln (8, 9) zur Aufnahme des einzusetzenden Stabes (19), einen an das andere Ende (3) angrenzenden Bereich zur Aufnahme des Kopfes (15) und
ein auf den Stab (19) bzw. auf den Kopf (15) Druck ausübendes Element (22, 17) aufweist,
**dadurch gekennzeichnet, daß**
der Gewindeabschnitt (13) und der Kopf (15) als separate Teile ausgebildet sind und daß
der Kopf (15) auf seiner dem Gewindeabschnitt (13) zugewandten Seite einen federnd nachgebenden Rand aufweist, wobei durch einen vom ersten Ende (2) her auf den Kopf (15) ausgeübten Druck der Kopf durch den federnden Rand einerseits mit dem Schaft verklemmt und gleichzeitig der Kopf in seiner Drehstellung arretiert wird.

2. Verankerungselement nach Anspruch 1, **dadurch gekennzeichnet, daß** der Gewindeabschnitt (13) an dem kopfseitigen Ende einen Schaft (14) aufweist.

3. Verankerungselement nach einem der Ansprüche 1 bis 3, **dadurch gekennzeichnet, daß** der dem Gewindeabschnitt zugewandte Rand (34) eine oder mehrere zur Symmetrieachse (4) parallel gerichtete und umfangsmäßig verteilte Durchbrechungen bzw. Ausnehmungen (28, 29, 33) aufweist.

4. Verankerungselement nach Anspruch 4, **dadurch gekennzeichnet, daß** eine Durchbrechung (33) sich in Richtung parallel zur Symmetrieachse (4) gesehen über die gesamte Wandlänge erstreckt.

5. Verankerungselement nach einem der Ansprüche 1 bis 5, **dadurch gekennzeichnet, daß** der Kopf (15) eine zur Symmetrieachse koaxiale Bohrung (27) aufweist.

6. Verankerungselement nach Anspruch 6, **dadurch gekennzeichnet, daß** die Bohrung (27) zylindrisch ausgebildet ist.

7. Verankerungselement nach einem der Ansprüche 2 bis 7, **dadurch gekennzeichnet, daß** der Schaft (14) eine rauhe Oberfläche aufweist.

8. Verankerungselement nach einem der Ansprüche 2 bis 8, **dadurch gekennzeichnet, daß** der Schaft (14) mehreckig ausgebildet ist.

9. Verankerungselement nach einem der Ansprüche 1 bis 6, **dadurch gekennzeichnet, daß** der Kopf (15) in der Bohrung ein Innengewinde und der Schaft (35) ein dazu passendes Außengewinde aufweist.

10. Verankerungselement nach einem der Ansprüche 1 bis 6, **dadurch gekennzeichnet, daß** der Kopf (15) in der Bohrung in Umfangsrichtung gewellt ausgebildet ist und der Schaft (37) an seiner Außenseite eine entsprechende Wellung aufweist.

## Claims

1. Anchoring element with a screw (12) comprising a shank having a threaded section (13) and a head (15) designed as a spherical segment-shaped section, and with a receiving portion (1) for connecting the screw (12) to a rod (19), wherein the receiving portion (1) comprises
a first end (2) and a second end (3) opposite the latter, a longitudinal axis (4) passing through the two ends (2, 3),
a bore (5) coaxial with the longitudinal axis (4), a first region adjoining the first end (2) with an essentially U-shaped cross-section (7) with two free arms (8, 9) comprising a thread for receiving the rod (19) to be inserted, a region adjoining the other end (3) for receiving the head (15), and
an element (22, 17) which exerts pressure on the rod (19) or on the head (15), **characterised in that** the threaded section (13) and the head (15) are designed as separate parts and **in that** the head (15) comprises a spring-resilient edge on its side facing towards the threaded section (13), wherein, due to a pressure exerted on the head (15) from the first end (2), the head is on the one hand clamped to the shank by the resilient edge and at the same time the head is locked in its rotational position.

2. Anchoring element according to claim 1, **characterised in that** the threaded section (13) comprises a shank (14) at the head end.

3. Anchoring element according to either of claims 1 and 2, **characterised in that** the edge (34) facing towards the threaded section comprises one or more apertures or recesses (28, 29, 33) which are directed parallel to the axis of symmetry (4) and distributed circumferentially.

4. Anchoring element according to claim 3, **characterised in that** an aperture (33) extends over the whole wall length, seen in a direction parallel to the axis of symmetry (4).

5. Anchoring element according to any of claims 1 to 4, **characterised in that** the head (15) comprises a bore (27) coaxial with the axis of symmetry.

6. Anchoring element according to claim 5, **characterised in that** the bore (27) is cylindrical.

7. Anchoring element according to any of claims 2 to 6, **characterised in that** the shank (14) comprises a rough surface.

8. Anchoring element according to any of claims 2 to 7, **characterised in that** the shank (14) is polygonal.

9. Anchoring element according to any of claims 1 to 5, **characterised in that** the head (15) comprises an internal thread in the bore and the shank (35) comprises an external thread mating therewith.

10. Anchoring element according to any of claims 1 to 5, **characterised in that** the head (15) is corrugated in the circumferential direction in the bore and the shank (37) comprises a corresponding corrugation on its outer side.

## Revendications

1. Élément d'ancrage avec une vis (12), présentant une tête (15) réalisée avec un tronçon fileté (13) et un tronçon en forme de segment de sphère, et une partie de logement (1) pour relier la vis (12) à une barre (15), la partie de logement (1) présentant :
une première extrémité (2) et une deuxième extrémité (3), opposée à celle-ci, un axe longitudinal (4) passant par les deux extrémités (2, 3),
un perçage (5) coaxial par rapport à l'axe longitudinal (4), une première zone limitrophe à la première extrémité (2), avec une section transversale (7) sensiblement en forme de U, avec deux branches (8, 9) libres, présentant un filetage, pour recevoir la barre (19) à insérer, une zone limitrophe à l'autre extrémité (3), pour recevoir la tête (15), et un élément (22, 17) exerçant une pression sur la barre (19) ou sur la tête (15),
**caractérisé en ce que**
le tronçon fileté (13) et la tête (15) sont réalisés sous forme de parties séparées, et **en ce que**
la tête (15), sur son côté tourné vers le tronçon fileté (13), présente un bord déformable élastiquement, où,
par une pression exercée par la première extrémité (2) sur la tête (15), la tête étant serrée par le bord élastique, d'une part, avec la tige et, simultanément, la tête étant arrêtée à sa position en rotation.

2. Élément d'ancrage selon la revendication 1, **caractérisé en ce que** le tronçon fileté (13) présente une tige (14) à l'extrémité située côté tête.

3. Élément d'ancrage selon l'une des revendications 1 à 2, **caractérisé en ce que** le bord (34), tourné vers le tronçon fileté, présente un ou plusieurs passages ou évidements (28, 29, 33), orientés parallèlement à l'axe de symétrie (4) et répartis en périphérie.

4. Élément d'ancrage selon la revendication 3, **caractérisé en ce qu'**un passage (33) s'étend sur toute la longueur de paroi, en observant dans une direction parallèle à l'axe de symétrie (4).

5. Élément d'ancrage selon l'une des revendications 1 à 4, **caractérisé en ce que** la tête (15) présente un perçage (27) coaxial à l'axe de symétrie.

6. Élément d'ancrage selon la revendication 5, **caractérisé en ce que** le perçage (27) est cylindrique.

7. Élément d'ancrage selon l'une des revendications 2 à 6, **caractérisé en ce que** la tige (14) présente une surface rugueuse.

8. Élément d'ancrage selon l'une des revendications 2 à 7, **caractérisé en ce que** la tige (14) est de configuration polygonale.

9. Élément d'ancrage selon l'une des revendications 1 à 6, **caractérisé en ce que** la tête (15) présente, dans le perçage, un filetage intérieur, et la tige (35) présente un filetage extérieur ajusté à celui-ci.

10. Élément d'ancrage selon l'une des revendications 1 à 6, **caractérisé en ce que** la tête (15) est ondulée en direction périphérique dans le perçage, et la tige (37) présente une ondulation correspondante sur sa face extérieure.
